# EUROPÄISCHE PATENTSCHRIFT

(11) **EP 0 522 355 B1**
(45) Veröffentlichungstag und Bekanntmachung des Hinweises auf die Patenterteilung: **08.11.1995**
(21) Anmeldenummer: 92110624.1
(22) Anmeldetag: 24.06.1992
(51) Int. Cl.: A61B 17/60

(54) **Klemmkupplung zum Verbinden von Knochenschrauben mit einem äusseren Fixateur**
Clamping joint for connecting bone screws to an external fixator
Accouplement à serrage pour rattacher des vis à os à un fixateur externe

(30) Priorität: 12.07.1991 DE 9108566 U
(43) Veröffentlichungstag der Anmeldung: 13.01.1993
(73) Patentinhaber: Pennig, Dietmar, Dr. med., 50935 Köln (DE)
(72) Erfinder: Pennig, Dietmar, Dr. med., 50935 Köln (DE)
(74) Vertreter: Habbel, Hans-Georg, Dipl.-Ing.

(56) Entgegenhaltungen:
- EP-A- 0 011 258
- WO-A-90/07305
- GB-A- 2 229 096

## Beschreibung

Die Erfindung bezieht sich auf eine Klemmkupplung gemäß dem Oberbegriff des Hauptanspruches.

Aus der US-PS 43 12 336 ist ein äußerer Fixateur bekannt, dessen zentrales Teil jeweils endseitig Klemmkupplungen aufweist, die einerseits zur Aufnahme und Festlegung von Knochenschrauben oder Pins ausgebildet sind, andererseits über ein Kugelgelenk und einen Bajonettverschluß oder Schraubverschluß an den zentralen Teil anschließen, so daß dadurch die Knochenschrauben mit dem zentralen Teil des Fixateurs verbunden werden. Die endseitig angeordneten Kugelgelenkanschlüsse können dabei gegenüber der Achse des zentralen Teiles des Fixateurs um etwa 40 bis 45^{o} abgewinkelt werden, was in einigen Fällen unzureichend ist.

Aus der GB-A-22 29 096 ist eine äußere Fixiervorrichtung insbesondere für Becken bekanntgeworden, bei welcher das zentrale Teil zwischen den beiden endseitigen Klemmkupplungen zusätzlich noch um eine Achse geschwenkt werden kann. Zu dieser Einrichtung wird ausdrücklich darauf hingewiesen, daß die Drehbewegung zwischen den beiden Mittel teilen in einer einzigen genau definieten Ebene erfolgen soll, und durch diese bekannte Anordnung wird der Abwinkelungsgrad der Anschlußmöglichkeit der Klemmkupplungen an dem Mittelteil nicht vergrößert.

Der Erfindung liegt also die Aufgabe zugrunde, die gattungsbildende Klemmkupplung dahingehend zu verbessern, daß ohne großen Aufwand auch größere Abwinkelungen zwischen dem zentralen Teil des Fixateurs und der eigentlichen Klemmkupplung möglich sind.

Diese der Erfindung zugrundeliegende Aufgabe wird dadurch gelöst, daß ein Drehgelenk zwischen dem Kugelgelenkanschluß und der Halbschale eingeschaltet wird, die den eigentlichen Kugelgelenkanschluß trägt.

Durch diese Anordnung wird nunmehr eine Abwinkelung ermöglicht, die größer als 90^{o} ist, wobei durch die Zwischenschaltung des Kugelgelenks zwischen die Befestigungsmuffe des Bajonettverschlusses und die Halbschale auch beliebige Ausrichtungen möglich sind.

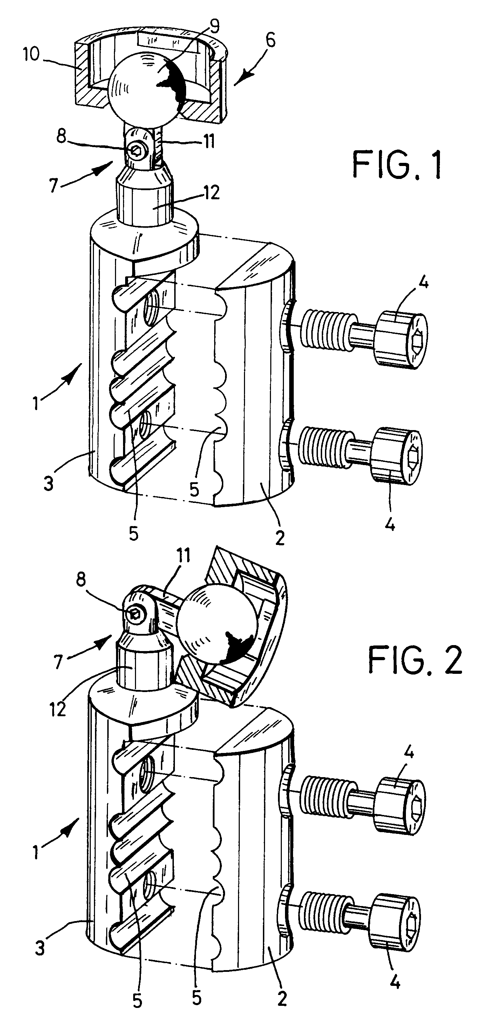

Ein Ausführungsbeispiel der Erfindung wird nachfolgend anhand der Zeichnung erläutert. Die Zeichnung zeigt in
- Fig. 1: in einer auseinandergezogenen Darstellung eine Klemmkupplung und in
- Fig. 2: eine Verdeutlichung der Abwinkelung.

In der Zeichnung ist eine Klemmkupplung 1 dargestellt, die im wesentlichen aus zwei Halbschalen 2 und 3 besteht, wobei die Halbschalen 2 und 3 mittels Schrauben 4 aufeinander festgelegt werden können. In den Halbschalen 2 und 3 sind Aufnahmen 5 vorgesehen, die der Aufnahme und Festlegung von Knochenpins, Schrauben od. dgl. dienen. Die Halbschale 2, 3 trägt fest einen Kugelgelenkanschluß 6 für einen Bajonettverschluß oder Schraubverschluß, mit dem diese Klemmkupplung 1 an das äußere Ende eines an sich bekannten äußeren Fixateurs angeschlossen werden kann. Der Kugelgelenkanschluß 6 besteht aus der Kugel 9 und einem Ring 10, der dem Anschluß an den Fixateur dient. Während im Stand der Technik die Kugel 9 des Kugelgelenkanschlusses 6 unmittelbar und starr an die Halbschale 2, 3 anschließt, ist bei der dargestellten Ausführungsform zwischen diesen beiden Bauteilen 3 und 6 ein Drehgelenk 7 eingeschaltet, dessen Gelenkachse bei 8 erkennbar ist, die gleichzeitig als "Feststeller" ausgebildet ist. An die Gelenkachse 8 schließt die Kugel 9 über einen Zapfen 11 und die Halbschale 3 über einen Bund 12 an, wobei die Gelenkachse 8 mit Gewinde ausgerüstet ist und dadurch der Zapfen 11 mit dem Bund 12 verklemmbar ist. Durch diese Anordnung ist es möglich, den eigentlichen Kugelgelenkanschluß 6 gegenüber der Klemmschale 3 um z. B. 90^{o} zu schwenken und festzustellen, so daß dadurch der Einsatzbereich der Klemmkupplung erhöht wird.

Zudem kann natürlich wie im Stand der Technik der Ring 10 auf der Kugel 9 verschwenkt werden.

## Patentansprüche

1. Klemmkupplung (1) zur Festlegung von Knochenschrauben, Pins od. dgl. und zum Verbinden derselben mit einem äußeren Fixateur, bestehend aus zwei mittels Schrauben (4) verbindbaren, und Aufnahmen (5) für die Knochenschrauben od. dgl. aufweisenden Halbschalen (2, 3) und einem an einer Halbschale befestigten Kugelgelenkanschluß (6), gekennzeichnet durch ein Drehgelenk (7) zwischen der den Kugelgelenkanschluß (6) tragenden Halbschale (3) und dem Kugelgelenkanschluß (6).

2. Klemmkupplung nach Anspruch 1, dadurch gekennzeichnet, daß das Drehgelenk (7) arretierbar ist.

## Claims

1. A compression coupling (1) for fixing bone screws, pins or the like and connecting them with an external fixer, consisting of two half-shells (2, 3), connectable by means of screws (4) and comprising receptacles (5) for the bone screws or the like, and of a ball-and-socket joint connection (6) fastened to one half shell, characterized by a swivel joint (7) between the half shell (3) carrying the ball-and-socket joint connection (6) and the ball-and-socket joint connection (6).

2. A compression coupling according to claim 1, characterized in that the swivel joint (7) is arrestable.

## Revendications

1. Accouplement (1) pour fixer des vis à os, des broches ou similaires et relier celles-ci à un fixateur externe, composé de deux demi-coques (2, 3) qui peuvent être reliées au moyen de vis (4) et qui présentent des logements (5) pour les vis à os ou similaires et d'un joint à rotule (6) fixé à une demi-coque, caractérisé en ce qu'il comporte une articulation rotative (7) entre la demi-coque (3) qui porte le joint à rotule (6) et le joint à rotule (6).

2. Accouplement à serrage selon la revendication 1, caractérisé en ce que l'articulation rotative (7) peut être arrêtée.
